Europäisches Patentamt

(19) European Patent Office

Office européen des brevets

(11) EP 1 063 968 B1

(12) EUROPEAN PATENT SPECIFICATION

(45) Date of publication and mention
of the grant of the patent:
**28.07.2004 Bulletin 2004/31**

(51) Int Cl.[7]: **A61K 9/00**, A61K 31/135,
A61K 47/26, A61K 9/14,
A61K 9/16, C07H 3/04

(21) Application number: **99917843.7**

(22) Date of filing: **24.03.1999**

(86) International application number:
**PCT/EP1999/001967**

(87) International publication number:
**WO 1999/048476 (30.09.1999 Gazette 1999/39)**

(54) **IMPROVED COMPOSITIONS FOR INHALATION**

VERBESSERTE INHALATIONSPRÄPARATE

COMPOSITIONS AMELIOREES POUR INHALATIONS

(84) Designated Contracting States:
**AT BE CH CY DE DK ES FI FR GB GR IE IT LI LU
MC NL PT SE**
Designated Extension States:
**AL LT LV MK RO SI**

(30) Priority: **26.03.1998 GB 9806462**

(43) Date of publication of application:
**03.01.2001 Bulletin 2001/01**

(73) Proprietor: **GLAXO GROUP LIMITED**
**Greenford, Middlesex UB6 ONN (GB)**

(72) Inventors:
• **LARHRIB, El Hassane**
**150 Stamford Street London, SE1 8WA (GB)**
• **MARRIOTT, Christopher**
**150 Stamford Street London, SE1 8WA (GB)**
• **MARTIN, Gary Peter**
**London SW3 6LX (GB)**
• **PRITCHARD, John Nigel**
**Middlesex UB11 1BT (GB)**
• **ZENG, Xian Ming**
**London SW3 6LX (GB)**

(74) Representative:
**Hammett, Audrey Grace Campbell et al
Glaxo Wellcome plc,
Glaxo Wellcome House,
Berkeley Avenue
Greenford, Middlesex UB6 0NN (GB)**

(56) References cited:
EP-A- 0 093 538       EP-A- 0 542 314
WO-A-95/01324        WO-A-97/48413
WO-A-98/17676        GB-A- 2 157 291

• M. AKBARIEH AND R. TAWASHI: "Morphic
features of solid particles after micronization in
the fluid energy mill" INTERNATIONAL
JOURNAL OF PHARMACEUTICS, vol. 35, no. 1-2,
1987, pages 81-89, XP002108926
• "Handbook of Pharmaceutical Excipients" 1988
, AMERICAN PHARMACEUTICAL
ASSOCIATION/PHARMACEUTICAL SOCIETY
OF GREAT BRITAIN XP002111136 see page 93 -
page 98; claims 1,4,7,12,14-16; figures 6,7; table
1 see page 153 - page 162; claims 1,4,7,12,14-16;
figures 6,7; table 1 see page 177 - page 180;
claims 1,4,7,12,14-16; figures 6,7; table 1 see
page 304 - page 308; claims 1,4,7,12,14-16;
figures 6,7; table 1 see page 346 - page 347;
claims 1,4,7,12,14-16; figures 6,7; table 1
• K. DANJO ET AL.: "Effect of Particle Shape on
the Compaction and Flow Properties of
Powders" CHEMICAL & PHARMACEUTICAL
BULLETIN, vol. 37, no. 11, 1989, pages
3070-3073, XP002108927

**Description**

The present invention relates to improved pharmaceutical compositions for inhalation, and the use of elongated drug and/or carrier particles therein.

Numerous medicaments, especially those for the treatment of respiratory conditions such as asthma, are administered by inhalation. Since the drug acts directly on the target organ much smaller quantities of the active ingredient may be used, thereby minimising any potential side effects caused as a result of systemic absorption. The efficacy of this route of administration has been limited by the problems encountered in making appropriate and consistent dosages available to the lungs. The delivery systems currently available are pressurised metered dose inhalers, nebulisers and dry powder inhalers.

Metered dose inhalers require good co-ordination of actuation and inhalation in order to achieve consistent dose administration; this co-ordination may be difficult for some patients. Nebulisers are effective but are relatively expensive and bulky and as a result are mainly used in hospitals. A variety of dry powder inhalers have been developed and, since dry powder inhalers rely on the inspiratory effect of the patient to produce a fine cloud of drug particles, the co-ordination problems associated with the use of metered dose inhalers do not apply.

It has been found that medicaments for administration by inhalation should be of a controlled particle size in order to achieve maximum penetration into the lungs, preferably in the range of 1 to 10 micrometers in diameter. Unfortunately, powders in this particle size range, for example micronised powders, have a high bulk volume and have very poor flow characteristics due to the cohesive forces between the individual particles. These characteristics create handling and metering difficulties during manufacture of the medicament powder and, most importantly, adversely affect the accurate dispensing of the powder within the inhalation device. A number of proposals have been made in the literature to improve the fluidity of dry powder pharmaceutical formulations.

GB1520248 describes the preparation of soft pellets of finely powdered sodium cromoglycate which have satisfactory fluidity within the reservoir of the inhaler device but have sufficiently low internal coherence to break up into finer particles of medicament when introduced into the turbulent air stream in the mouthpiece of the device. Numerous other published patent applications suggest the use of carrier materials, for example GB1402423, particularly of coarser carriers with particles having sizes falling within a given range, for example GB1242211, GB1381872, GB1410588, GB1478020 and GB1571629. WO87/05213 describes a carrier which comprises a conglomerate of one or more solid water-soluble diluents and a lubricant, EP0260241 describes a lipid-based dry powder composition, and US5143126 describes a method of preparing flowable grain agglomerations of formoterol and lactose. Unfortunately the selection of the particle size of the drug and excipient and of the ratio of drug to excipient inevitably involves a compromise between adequate bulk and flow properties for metering and the desired redispersability of fine particle drug in the inhaled air flow.

Surprisingly, we have now found that crystals of lactose monohydrate having high elongation ratios may, when employed in powder compositions suitable for inhalation, increase the fine particle fraction (FPF) of the drug, compared to crystalline lactose monohydrate with lower elongation ratios (see Table 6). Since formulations that produce a higher FPF can be expected to deliver a higher fraction of drug to the lower airways than those which produce a lower FPF, crystals of lactose monohydrate with a higher elongation ratio provide advantageous inhalation compositions.

Accordingly, the present invention provides a pharmaceutical composition for administration via inhalation, comprising drug selected from the group consisting of salbutamol, fluticasone propionate, beclomethasone dipropionate, formotorol, budesonide, ipratropium, oxitropium or a physiologically acceptable salt or solvate thereof and lactose monohydrate crystals having an elongation ratio in the range 1.55 - 2.20.

Preferably the average size of the particles of the carrier is in the range 5 to 1000 micrometers, more preferably in the range of 30 to 250 micrometers, and most preferably in the range 50 to 100 micrometers. Typically at least 95% of the particles will be of a size which falls within this range.

Preferably the elongation ratio is the range 1.60 - 2.10.

It will be appreciated by those skilled in the art that the powder compositions according to the invention may, if desired, contain a combination of two or more active ingredients.

Preferably salbutanol is in the form of a sulphate salt.

The final powder composition desirably contains 0.1 to 90% w/w, preferably 0.5 to 75% w/w, especially 1-50% w/w, of medicament relative to the weight of the carrier particles.

Once formed, the carrier particles may be admixed with microfine particles of one or more drugs, optionally together with one or more conventional pharmaceutically acceptable ingredients, using conventional techniques to prepare the powder compositions according to the invention.

The powder compositions according to the invention optionally contain one or more conventional pharmaceutically acceptable ingredients such as diluents and flavouring agents. The particle size of any such ingredients will preferably be such as to substantially prevent their inhalation into the bronchial system upon administration of the powder composition, desirably in the range of 50 to 1000 micrometers.

The final powder composition desirably contains 0.1 to 90% w/w, preferably 1 to 20% w/w of medicament and 10 to 99.9% w/w, preferably 50 to 99% w/w of carrier particles.

Crystals with a controlled elongation ratio may be prepared by various methods, for example by super critical fluid crystallisation, such as that described in WO95/01324, by recrystallisation as described hereinafter, or by growing the crystals in a variable-viscosity medium as described hereinafter.

Elongated crystals may be prepared by recrystallisation from conventional solvents under controlled conditions. In order to obtain crystals of a suitable size and shape for inhalation (so as to avoid the need for micronisation), the substance to be crystallised should be dissolved in a solvent and the solution added to a second solvent, in which the substance is not soluble but which is miscible with the first solvent. After adding the solution to the second solvent, the substance crystallises so rapidly that only small crystal nuclei are prepared.

Stirring is not required in this technique.

For example, elongated salbutamol sulphate crystals may be prepared by adding an aqueous solution of salbutamol sulphate to absolute ethanol.

Using this method, we have surprisingly found that the size and shape of the crystals can be predictably controlled by varying the concentration of the second solvent. For example, the crystal shape of lactose particles obtained by adding acetone to an aqueous solution of lactose moves through tomahawk shape at 65-70% acetone, to needle shaped at 75% acetone and above. The particle size decreases with increasing acetone concentration, and thus it is possible to obtain the desired elongation ratio by selecting the appropriate concentration of acetone.

Elongated crystals may be prepared in a viscosity-variable medium by :

a) dissolving the substance to be crystallised in a medium wherein the viscosity of the medium can be adjusted;

b) applying a means for adjusting the viscosity of the medium until a gel with an apparent viscosity in the range 25 to 90 Pa.s at a shear rate of $1s^{-1}$ is reached;

c) allowing crystal growth;

d) applying a means for adjusting the viscosity of the medium until a fluid with an apparent viscosity less than 25 Pa.s at a shear rate of $1s^{-1}$ is reached; and

e) harvesting the crystals.

The means for adjusting the viscosity of the medium may be, for example temperature change, ultrasound, thixotropicity, electro-rheology (application of an electric current), mechanical shear, chemical additive (for example sodium chloride or ethanol), or pH change. Preferably, the means for adjusting the viscosity of the medium is pH change.

The medium may be in the form of an aqueous or organic solution of a polymer. Preferably, the medium is an aqueous solution of a polymer.

Preferably the medium used to prepare the crystals intended to be used as a drug or carrier in dry powder inhalation formulations has to meet at least the following criteria. First, the medium should be suitable for use as a pharmaceutical ingredient for internal usage. Second, the medium should preferably be capable of being efficiently removed from the surface of the crystals so as not to affect any physico-chemical properties of the crystals and, most importantly, to minimise the possibility of introducing such a compound to the respiratory tract. Third, the consistency or viscosity of the medium can be controlled such that after crystallisation, the bulk of crystals can be harvested easily without any vigorous treatment that might change the morphology of the crystals.

Preferably the polymer which comprises the medium is a Carbomer. Carbomers, a group of polyacrylic acid polymers cross-linked with either allylsucrose or allyl ethers of pentaerythritol, provide a medium that meets the aforementioned criteria. Carbomers have been widely used as suspending agents; emulsifying agents or tablet binders in pharmaceutical industry. Carbomer gels have also been employed as bioadhesive vehicles for mucoadhesive drug delivery formulations to prolong drug residence at the application sites. The viscosity of Carbomer gels is known to be dependent upon the polymer concentration (Barry and Meyer, Int. J.Pharm. 1979; 2; 1-25) and therefore, it is possible to obtain a minimal viscosity that can suspend the crystals without substantially inhibiting crystal growth. The viscosity of Carbomer gel changes reversibly with the pH value of the solution (Barry and Meyer, Int. J. Pharm, 1979; 2; 27-40). Carbomers disperse in water to form acidic colloidal solutions of low viscosity which, when neutralised, produce highly viscous gels. The viscosity reaches a maximum at pH 6-11 but is considerably reduced if the pH is less than 3 or greater than 12. Therefore, the crystallisation can be carried out in a neutralised Carbomer gel. After which, the gel can be converted to a fluid by acidification such that the crystals may be readily harvested. In order to remove the medium from the surface of the crystals, a solvent in which the Carbomer is soluble but the crystals are insoluble is required. Carbomers are soluble in both ethanol and glycerine, whereas the preferred crystals, lactose, are insoluble in these solvents. Therefore, any adsorbed Carbomer residue on lactose crystals may be easily removed by washing the crystals with either ethanol or glycerine without substantially changing the morphology of the crystals.

The pH of the medium may be adjusted by the addition of an aqueous base, for example it may be raised by the addition of aqueous sodium hydroxide solution, or it may be lowered by the addition of an aqueous acid, for example it may be lowered by the addition of hydrochloric acid.

Most preferably the medium is a Carbopol 934™ gel. Preferably the gel is an aqueous dispersion of Carbopol 934™ at a concentration of at least 0.4% w/w. Preferably, the concentration of Carbopol 934™ is in the range 0.4-0.8% w/w.

Preferably, the pH of the Carbopol 934™ gel is initially adjusted to be in the range pH 6.5-7.5, providing an apparent viscosity in the range 25-90 Pa.s depending on the concentration.

Preferably, after the crystal growth the pH of the Carbopol 934™ gel is adjusted to be in the range pH 3-3.5, providing a fluid.

It will be understood by those skilled in the art that other Carbomers may be used in the present invention, with concentration and pH parameters determinable by methods known in the art.

Preferably crystal growth is monitored, for example by use of an optical microscope, until the majority of the crystals have grown to a size in the range 50-125 μm, more preferably 63-90 μm.

The substance to be crystallised may be a drug substance or a carrier for drug particles, suitable for use in an inhaled pharmaceutical composition, or may be, for example an additive for paint. Preferably, the substance to be crystallised is a water-soluble drug or a carrier.

The crystals may be harvested by standard techniques known in the art. For example, the crystals may be collected by filtration or by decanting the supernatant and drying the crystals. Preferably, the harvested crystals are washed in a solvent in which the medium is soluble and the crystals are insoluble.

When the medium is a Carbomer, preferably the harvested crystals are washed in a solvent in which the Carbomer is soluble and the crystals are insoluble, for example ethanol or glycerine.

Crystals, for example lactose monohydrate crystals, prepared according to the process described above, have a significantly higher mean elongation ratio and "surface factor" (see Table 3), and an improved degree of crystallinity (see Table 4) and flowability (significantly smaller angle of slide, see Table 5) than crystals prepared by a standard constant stirring technique.

The compositions according to the invention may conveniently be filled into a bulk storage container, such as a multi-dose reservoir, or into unit dose containers such as capsules, cartridges or blister packs, which may be used with an appropriate inhalation device, for example as described in GB2041763, WO91/13646, GB1561835, GB2064336, GB2129691 or GB2246299. Such inhalers which contain a composition according to the invention are novel and form a further aspect of the invention. The compositions of the invention are particularly suitable for use with multi-dose reservoir-type inhaler devices in which the composition is metered, e.g. by volume from a bulk powder container into dose-metering cavities. The lower limit of powder delivery which may be accurately metered from a multi-dose reservoir-type inhaler device is in the region of 100 to 200 micrograms. The formulations of the present invention are therefore particularly advantageous for highly potent and hence low dose medicaments which require a high ratio of excipient for use in a multi-dose reservoir-type device.

Dry powder inhalers are designed to deliver a fixed unit dosage of medicament per actuation, for example in the range of 10 to 5000 micrograms medicament per actuation, preferably 25 to 500 micrograms.

Administration of medicament may be indicated for the treatment of mild, moderate or severe acute or chronic symptoms or for prophylactic treatment. It will be appreciated that the precise dose administered will depend on the age and condition of the patient, the particular medicament used and the frequency of administration and will ultimately be at the discretion of the attendant physician. When combinations of medicament are employed the dose of each component of the combination will in general be that employed for each component when used alone. Typically, administration may be one or more times, for example from 1 to 8 times per day, giving for example 1, 2, 3 or 4 unit doses each time.

Thus, for example, each actuation may deliver 25 micrograms salmeterol, 100 micrograms salbutamol, 25, 50, 125 or 250 micrograms fluticasone propionate or 50, 100, 200 or 250 micrograms beclomethasone dipropionate.

The present invention is illustrated by the following Examples.

## Examples

### Example 1

#### Preparation of lactose monohydrate crystals using the constant stirring technique

<u>One-step crystallisation from aqueous solution</u> - A predetermined amount of lactose (Lactochem™, Borculo Whey Ltd., Chester, UK) was dissolved in 100 ml distilled water at 80°C. After filtration through a Whatman filter paper (<0.45 µm), the solution was transferred to a 150 ml glass beaker which had been placed in either an ice bath or a water bath at 40°C. The solution was stirred at 500 rpm (Heidolph Overhead Stirrer, Fisons Laboratory Instruments, UK) with a 4 blade (1x3 cm) stirrer which was situated 2 cm above the bottom of the container. After the crystallisation was allowed to continue for a predetermined period of time, the crystals were filtered and washed sequentially with 60% (v/v) and absolute ethanol, respectively. The crystals were allowed to dry at room temperature overnight before drying in a vacuum oven at 70°C for 3 h. After a small amount of sample (about 0.5 g) was taken from each batch of lactose for the measurement of particle size, shape and surface smoothness, the remaining lactose crystals were poured into a 90 µm sieve which had been placed upon a 63 µm sieve. The particles were then sieved manually and slowly for 1 h so as not to rupture any crystals. The particles were divided into 3 size fractions (< 63, 63-90 and >90 µm), which were collected and weighted separately. The lactose crystals thus obtained were transferred to a sealed vial and placed into a desiccator over silica gel until required for further investigation. The samples obtained are given in Table 1 below.

Two-stage crystallisation from aqueous solution - Lactochem™ lactose (200g) was dissolved in 200ml distilled water at about 90°C. The solution (about 320ml) was filtered while still hot through a Whatman filter paper (0.45µm). It was then transferred to a 500ml glass beaker and stirred at 500 rpm with a 4 blade (1x3 cm) stirrer which was situated 2cm above the bottom of the container. Lactose was then allowed to crystallise under constant stirring at room temperature at 500 rpm for 2.5 h. The crystals (A) were filtered and the mother liquor was placed back into the beaker and allowed to crystallise further for 16 h to obtain crystals (B). Batches A and B were washed with 60% (v/v) and absolute ethanol, respectively, and were allowed to dry at room temperature overnight. The lactose crystals were poured into a 90µm sieve which had been placed upon a 63µm sieve. The particles were then sieved manually and slowly for 1 h so as not to rupture any crystals. Batch (A) was classified into batches 13 and 14, which had a particle size range from 63-90µm and < 63µm respectively. Batch (B) was classified into batches 15 and 16, which had a particle size range from 63-90µm and < 63µm respectively. The crystals were then dried in a vacuum oven at 70°C for 3h. The lactose crystals thus obtained (batches 13 to 16) were transferred to a sealed vial and placed into a desiccator over silica gel until required for further investigation. The samples obtained are given in Table 1a below.

TABLE 1

| Batch No | Lactose (% w/w) | T (°C) | Time (h) | Diameter ($d_{sv}$) ± SD (µm) | % Particle (µm) | | | Shape |
|---|---|---|---|---|---|---|---|---|
| | | | | | < 63 | 63-90 | >90 | |
| 1 | 33 | 40 | 12 | 83.6 ± 12.8 | 13.9 | 45.8 | 40.3 | Tomahawk |
| 2 | 33 | 40 | 24 | 115.8 ± 14.6 | 5.6 | 15.1 | 79.3 | Tomahawk |
| 3 | 33 | 0 | 24 | 100.3 ± 18.9 | 15.2 | 17.2 | 67.6 | Irregular |
| 4 | 43 | 0 | 5 | 94.4 ± 13.4 | 19.6 | 21.8 | 56.6 | Irregular |
| 5 | 43 | 0 | 12 | 104.5 ± 14.8 | 14.9 | 23.2 | 61.9 | Irregular |
| 6 | 43 | 40 | 5 | 103.8 ± 20.6 | 14.4 | 21.6 | 64.0 | Tomahawk |
| 7 | 33 | 0 | 12 | 63.7 ± 9.4 | 33.0 | 40.0 | 26.8 | Irregular |
| 8 | 43 | 40 | 12 | 100.6 ± 15.3 | 24.5 | 17.9 | 57.6 | Pyramid |
| 9 | 50 | 40 | 3 | 88.8 ± 13.8 | 27.5 | 31.9 | 40.6 | Prism |
| 10 | 60 | 40 | 0.3 | 76.4 ± 15.7 | 33.8 | 46.3 | 19.9 | Elongated |
| 11 | 60 | 40 | 1.5 | 91.8 ± 17.9 | 26.3 | 27.6 | 46.1 | Elongated |

TABLE 1a

| Batch No | Diameter ($d_{sv}$) (µm) |
|---|---|
| 13 | 104.7 |
| 14 | 68.6 |
| 15 | 93.0 |

TABLE 1a   (continued)

| Batch No | Diameter ($d_{sv}$) (μm) |
|----------|--------------------------|
| 16 | 65.3 |

Example 2

Preparation of lactose monohydrate crystals using Carbomer gel

A predetermined amount of distilled water was agitated at about 500 rpm with a 4-bladed stirrer (1x3 cm) which was situated 2 cm above the bottom of a 500 ml beaker. The required amount of Carbopol 934™ (B F Goodrich Chemical Co., Cleveland, Ohio, USA) with an average molecular weight of approximately 3,000,000, was added into the vortex. When all the Carbopol was dispersed, the liquid was allowed to stand overnight in the dark so as to ensure maximum dissolution of the polymer. A cloudy, colloidal solution of low viscosity was obtained, the pH of which was about 3.2. The required amount of Lactochem™ lactose was then dissolved in the Carbopol solution at an elevated temperature (< 90°C, depending upon the final lactose concentrations) under constant stirring at 500 rpm to obtain a cloudy solution with a pH value of approximately 2.5. Sodium hydroxide solution (1 M) was then added dropwise to the solution, whilst stirring at about 800 rpm. The viscosity and clarity of the solution increased with pH, until it became a clear homogenous gel at approximately pH 4.5. After then, the mixer was not sufficiently powerful to disperse the gel and hence, the mixing was continued manually with a spatula. The addition of the neutralising agent (NaOH) was continued so as to obtain pH 7. The gel was then centrifuged at 3000 rpm for about 10 min so as to remove any entrapped air bubbles and insoluble particles. The gel was finally placed in the dark until the majority of the crystals had grown to the size range of 63-90 μm, which was estimated by an optical microscope, the gel was adjusted to pH 3-3.5 with hydrochloric acid (1 M) to obtain a fluid. The crystals were allowed to settle for about 10 min. After decanting the supernatant, the crystals were routinely washed with 60% ethanol twice and absolute ethanol three times. The crystals were finally allowed to dry at room temperature after which, a small amount of sample (about 0.5 g) was taken from each batch of lactose, the remaining lactose crystals were poured into a 90 μm sieve which had been placed upon a 63 μm sieve. The particles were then sieved manually and slowly for 1 h so as not to rupture any crystals. The particles were thus divided into 3 size fractions (< 63, 63-90 and > 90 μm) which were collected and weighted separately. The classified lactose crystals were dried in a vacuum oven at 70°C for 3 h before transferring to sealed vials, which were then placed in a desiccator over silica gel.

Crystallisations of the lactose from Carbopol 934™ gels were carried out under different conditions by means of altering the crystallisation time and the concentrations of either lactose or Carbopol gels (Table 2). Three batches of lactose crystals were prepared under each of the seven conditions listed in Table 2 but in each case the 3 batches were then mixed to prepare final batches of lactose, which were labelled as Car 1 to Car 7, respectively. The 63-90 μm fraction of batches Car 1 to Car 7 were labelled as C1 to C7, respectively. Lactose crystals from batch Car 1 were further classified into fractions < 63; 90-125 and > 125 μm, which in turn were labelled as C8; C9 and C10 respectively. Batch C7 was washed directly with 100% ethanol rather than pre-washing with 60% v/v ethanol as described above.

TABLE 2

| Batch No. | Lactose (%w/v) | Carbopol (% w/v) | Crystal time (h) | Mean Size (μm) | % Particle (μm) | | |
|-----------|----------------|------------------|------------------|----------------|-----|-----|-----|
| | | | | | <63 | 63-90 | > 90 |
| Car 1 | 43.0 | 0.6 | 72 | 105.4 | 5.8 | 35.4 | 58.8 |
| Car 2 | 43.0 | 0.3 | 24 | 87.9 | 10.3 | 56.5 | 33.2 |
| Car 3 | 33.0 | 0.3 | 24 | 76.5 | 12.2 | 68.7 | 19.1 |
| Car 4 | 50.0 | 0.4 | 48 | 116.3 | 8.2 | 12.6 | 79.2 |
| Car 5 | 50.0 | 0.6 | 72 | 114.2 | 1.4 | 22.3 | 76.3 |
| Car 6 | 38 | 0.4 | 72 | 93.3 | 8.5 | 53.5 | 38.0 |
| Car 7 | 38 | 0.4 | 48 | 75.4 | 15.6 | 73.2 | 11.2 |

Example 3

The shape factor (Scir), elongation ratio (E) and surface factor (Srec) of the samples was calculated in the following manner:

A small amount of lactose particles was scattered on a microscope slide using a small brush ensuring that the particles deposited separately. The slide was then mounted on an optical microscope (Labophot-2, Nikon, Japan) and the images of the particles were transferred to an IBM compatible computer through a Nikon camera. Particle images were analysed automatically using analySIS 2.0 (SIS Image Analysis GmbH, Germany) and the following descriptors were employed to quantify the morphology of lactose crystals:

$$\text{Shape factor} = S_{cir} = \frac{4 \, \Pi \, \text{area}}{\text{perimeter}^2}$$

$$\text{Elongation ratio} = E = \frac{\text{Length}}{\text{Width}}$$

$$\text{Surface factor} = S_{rec} = S_{cir} \times \frac{(1 + E)^2}{\Pi E}$$

All the particles that were projected onto the monitor were analysed and more than 100 particles were measured for each batch.

## TABLE 3

| Crystallisation with Constant Stirring | | | | Crystallisation in Carbopol 934™ gels | | | |
|---|---|---|---|---|---|---|---|
| Batch No. | $S_{cir}$ | E | $S_{rec}$ | Batch No. | $S_{cir}$ | E | $S_{rec}$ |
| 1 | 0.74 | 1.39 | 0.97 | C1 | 0.76 | 1.58 | 1.02 |
| 2 | 0.74 | 1.39 | 0.97 | C2 | 0.70 | 1.61 | 0.94 |
| 3 | 0.60 | 1.28 | 0.78 | C3 | 0.68 | 1.59 | 0.91 |
| 4 | 0.68 | 1.29 | 0.88 | C4 | 0.73 | 1.85 | 1.02 |
| 5 | 0.72 | 1.30 | 0.93 | C5 | 0.76 | 1.55 | 1.01 |
| 6 | 0.69 | 1.64 | 0.93 | C6 | 0.71 | 2.03 | 1.02 |
| 7 | 0.74 | 1.34 | 0.96 | C7 | 0.68 | 1.78 | 0.94 |
| 8 | 0.72 | 1.37 | 0.94 | | | | |
| 9 | 0.78 | 1.63 | 1.05 | | | | |
| 10 | 0.68 | 2.08 | 0.99 | | | | |
| 11 | 0.73 | 1.71 | 1.00 | | | | |
| 13 | 0.65 | 1.79 | 0.90 | | | | |
| 14 | 0.65 | 1.55 | 0.87 | | | | |
| 15 | 0.69 | 1.81 | 0.96 | | | | |
| 16 | 0.72 | 1.54 | 0.96 | | | | |

<u>Example 4</u>

<u>Degree of Crystallinity</u>

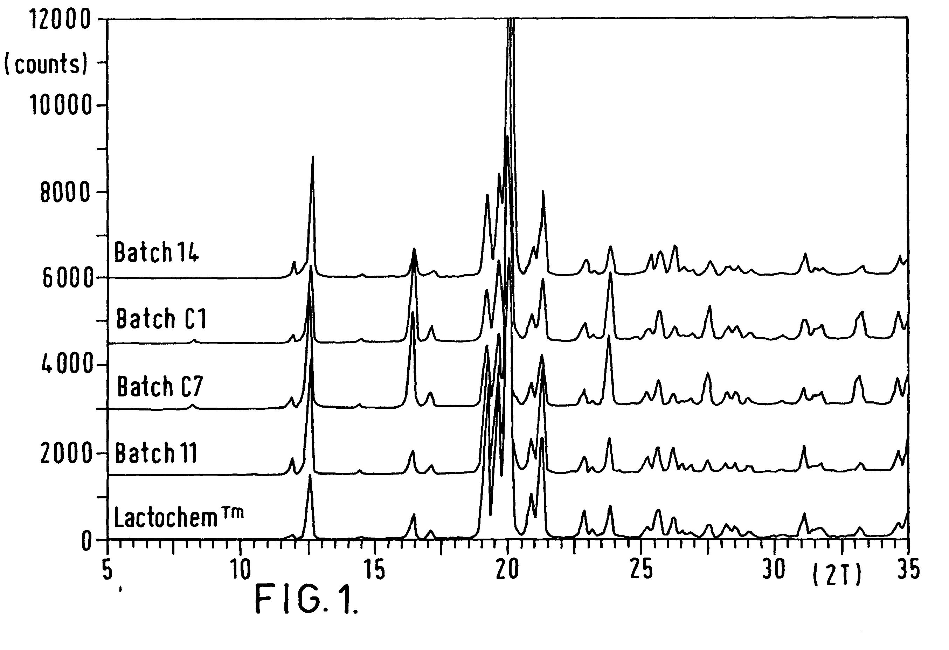

X-ray powder diffraction (XRPD) patterns for different batches of lactose were performed (Figure 1). All batches had similar XRPD patterns to $\alpha$-lactose monohydrate (Brittain *et al*, Pharm. Res. 1991, <u>8</u>, 963-973 and Sebhatu *et al*, int. J. Pharm. 1994, <u>104</u>, 135-144). However, different batches showed different peak intensities, which were indicative of different degrees of crystallinity of these lactose crystals.

X-ray powder diffractometry has been widely used to determine the degree of crystallinity of pharmaceuticals (Suryanarayanan, in Brittain HG (Ed), Physical Characterisation of Pharmaceutical Solids, Marcel Dekker, NY, 1995, 187-222). Some XRPD methods involve the demarcation and measurement of the crystalline intensity and amorphous intensity from the powder patterns (Nakai *et al*, Chem. Pharm. Bull. <u>30</u>, 1982, 1811-1818) whilst others employ an internal standard such as lithium fluoride to measure the crystallinity of drugs. Therefore, it is not possible to calculate the absolute degree of crystallinity by the XRPD patterns in Figure 1 since neither 100% amorphous lactose nor any internal standard was measured. However, since the degree of crystallinity is a function of either the integrated intensity (area under the curve) or the peak intensity (height), the relative degree of crystallinity of different samples of the same crystal forms may be compared by their peak intensity at the same diffraction angle. The relative degree of crystallinity (RDC) was defined as the ratio of the peak intensity of a given sample of a single polymorphic form to that of another specimen of the same polymorph which produced the greatest possible response (Ryan, J. Pharm. Sci. 75, 1986, 805-807). RDC may be employed to determine the rank order of crystallinity of different batches of lactose crystals. The integrated peak intensities at $2\theta$ = 12.5°, 16.5°, 23.8° and 27.5°, which are characteristic for $\alpha$-lactose monohydrate, were determined by measuring the areas under the curve of the X-ray diffraction profiles. The RDC was calculated by dividing the sum of the four integrated peak intensities of each batch by that of batch C7 since this batch produced the greatest trace of X-ray diffraction. It can be seen from Table 4 that the degree of crystallinity decreases in the order of batch C7 > batch C1 > Lactochem™ lactose > batch 11 > batch 14.

TABLE 4

| Estimates of the integrated peak intensities ($cm^2$) of XRDPs and the relative degree of crystallinity (RDC) of lactose crystals | | | | | |
|---|---|---|---|---|---|
| Angle ($2\theta$) | Lactochem™ | Batch 11 | Batch 14 | C1 | C7 |
| 12.5° | 0.72 | 0.70 | 0.41 | 0.58 | 0.81 |
| 16.5° | 0.11 | 0.88 | 0.10 | 0.67 | 0.68 |
| 23.8° | 0.16 | 0.11 | 0.16 | 0.45 | 0.40 |
| 27.5° | 0.04 | 0.07 | 0.07 | 0.19 | 0.17 |
| Sum | 1.03 | 0.96 | 0.74 | 1.89 | 2.06 |
| RDC (%) | 50.0 | 46.6 | 35.9 | 91.7 | 100 |

The lactose crystals prepared from Carbopol 934™ gels had a higher degree of crystallinity than lactose particles crystallised under conditions of constant mechanical agitation.

Example 5 - Flowability

The angle of repose ($\theta_r$) for batches of lactose crystals was measured (at least in triplicate) by pouring a sample of crystals into a copper tube (2.65 cm x 6.90 cm), which had been placed over a flat base with a diameter of 2.53 cm. After the powder heap reached a height of approximately 4 cm, the addition of powder was stopped and the copper tube was slowly lifted vertically off the base, on which a cone of powder was formed. The height of the cone was measured using a ruler and the $\theta_r$ calculated as:

$$\theta_r = \text{Tangent}^{-1}\left(\frac{hp}{r_b}\right)$$

where hp is the height (cm) of the powder heap and $r_b$ is the radius (cm) of the base.

The angle of slide ($\theta_s$) for batches of lactose crystals was measured, at least in triplicate, by placing lactose crystals (approximately 10mg) on a stainless steel plane (6.55 x 7.00 cm). The plane was tilted by screwing a spindle vertically upwards below the plane. When the majority of the powder started to slide, the angle between the tilted plane and the horizontal base, $\theta_s$, was directly read from a protractor. The results are listed in Table 5.

# TABLE 5

The angle of repose and angle of slide of different batches of lactose crystals
[mean (SD), n ≥ 3]

| Crystallisation with agitation | | | Crystallisation in Carbopol 934™ gels | | |
|---|---|---|---|---|---|
| Batch No. | $\theta_r$ (°) | $\theta_s$ (°) | Batch No. | $\theta_r$ (°) | $\theta_s$ (°) |
| 1 | 43 (1) | 50 (1) | C1 | 46 (1) | 48 (0) |
| 3 | 41 (1) | 47 (1) | C2 | 40 (0) | 43 (1) |
| 4 | 43 (1) | 50 (2) | C3 | 41 (2) | 45 (1) |
| 5 | | 46 (2) | C4 | 40 (1) | 45 (2) |
| 6 | 53 (1) | 62 (1) | C5 | 42 (2) | 48 (1) |
| 7 | 38 (0) | 43 (1) | C6 | 41 (0) | 43 (1) |
| 8 | 56 (2) | >90 | C7 | 43 (1) | 40 (1) |
| 9 | 37 (1) | 43 (1) | Lactochem™ | 48 (2) | 50 (1) |
| 10 | 34 (1) | 38 (1) | | | |
| 11 | 32 (1) | 34 (1) | | | |
| 13 | 58 (1) | 74 (1) | | | |
| 14 | 60 (0) | >90 | | | |
| 15 | 57 (2) | 71 (0) | | | |
| 16 | 59 (1) | >90 | | | |

Table 5 shows that different batches of lactose exhibited different degrees of both the angle of repose ($\theta_r$) and the angle of slide ($\theta_s$). Lactose particles from batches 10 and 11 produced significantly ($p < 0.01$) smaller values of $\theta_r$ or $\theta_s$ than the other batches of lactose, indicating that the former had higher flowability than the latter. The majority of lactose crystals from batches 10 and 11 had an elongated, cuboidal shape (Table 1). Elongated particles are known to build up open packings of high porosity. In flow, such particles tend to be oriented with their long axes in the direction of the flow and if such an orientation is achieved, these particles show less internal friction than more isometric particles (Neumann, Adv. in Pharm. Sci. 2, 1967, 181-221). Batches 14 and 16 produced the largest $\theta_r$ and these particles did not even slide off the plane that had been tilted to an angle of 90° to the horizontal, indicating that these two batches of lactose were highly cohesive and had poor flowability. This is likely to be attributable to the smaller mean diameter (approximately 65 μm) of batches 14 and 16 in comparison to the other batches of lactose (> 90 μm) since powders of smaller particle size are known to produce larger $\theta_r$ due to their internal cohesiveness (Neumann, Adv. in Pharm.

Sci. 2 1967, 181-221). Lactose particles prepared from Carbopol 934™ gels showed more consistent values of $\theta_r$ (40-46°) and $\theta_s$ (40-48°) in comparison to crystals prepared using agitation and this is likely to be due to more effective control of their particle morphology. Further, the crystals prepared from Carbopol 934™ gels appeared to have better flowability than the majority of the batches prepared under constant stirring since they had significantly (p < 0.01) smaller values of $\theta_s$ than the other batches of lactose (batches 1-8). The angle of repose differs from the angle of slide in that the former is determined by the least stable particles whilst the latter depends largely on the average conditions for the bulk of the powder (Hiestand, J. Pharm. Sci. 55, 1966, 1325-1344). Therefore, the angle of slide may correlate more closely with flow properties than the angle of repose.

Example 6 - Deposition profiles of salbutamol sulphate from different batches of lactose crystals

Salbutamol sulphate and lactose were mixed in a ratio of 1:67.5, w/w in accordance with the ratio employed in the commercial "Ventolin™" formulation. After drying in a vacuum over at 40°C for 12 h, micronised salbutamol sulphate with mass median diameter 2.0 µm (Glaxo Wellcome Group Ltd., Ware, UK) (25 mg), was weighed into a 10 ml stoppered sample vial to which had been added one spatula full of lactose crystals. The vial was stoppered and placed on a Whirlymixer for 5 s. Then, more lactose particles (similar to the amount of the blend) was added to the vial and the blend was mixed on a Whirlymixer for another 5 s. This process was repeated until all the lactose (1.750 g) had been incorporated into the salbutamol sulphate/lactose blend to obtain a ratio of drug to carrier of 1 : 67.5, w/w. The stoppered vials were then placed in a Turbula mixer (Glen Creston Ltd., Middx, UK) and mixed for 30 min. The samples were then stored in a vacuum desiccator over silica gel until further required.

Ten samples were taken randomly from each batch. The sample (approximately 33 mg) was weighed accurately and the amount of salbutamol sulphate was measured by HPLC. The coefficient of variation of the drug content was employed to assess the homogeneity of the mixtures.

Hard gelatin capsules (Size 3, Rotacapsule™, Glaxo Wellcome Group Ltd., Ware, UK) were filled with 33.0 ± 1.5 mg of the powder mixture so that each capsule contains 481 ± 22 µg salbutamol sulphate, which was the unit dose contained in a Ventolin Rotacap™. The filling was performed manually.

Ethyl paraben was dissolved in the mobile phase to produce a solution with a concentration of 4 µg ml$^{-1}$.

An accurately weighed amount of salbutamol sulphate (20.0 mg) was transferred to a 100 ml volumetric flask, dissolved in the internal standard solution, and made up to volume to obtain a concentration of 0.2 mg ml$^{-1}$ of salbutamol sulphate (solution A). 10.0 ml of solution A was pipetted into another 100 ml volumetric flask and diluted to volume with the internal standard solution to obtain a solution containing 20 µg ml$^{-1}$ salbutamol sulphate (solution B).

Aliquots of solution B (0.25, 0.50, 1.00, 2.00, 3.00, 4.00, 5.00, 6.00, 7.00 ml) were pipetted into 10 ml volumetric flasks and made up to volume using -the internal standard solution to obtain a series of the standard solutions which contained drug concentrations of 0.5, 1.0, 2.0, 4.0, 6.0, 8.0, 10, 12 and 14 µg ml$^{-1}$ respectively. These standard solutions were employed to construct a calibration curve of drug concentration against the peak area ratios of drug to internal standard. The calibration was prepared on a daily basis and a calibration curve with $r^2$ > 0.99 was considered acceptable.

Approximately 33 mg of the powder mixture was accurately weighed and dissolved in the internal standard solution. After the solution had been sonicated in a water bath for 30 min, it was filtered through a millipore filter (Whatman membrane filters, 0.45 µm, nylon, Whatman Lab. Division, Kent, UK). 30 µl of the filtrate was injected into the HPLC. No interference from the lactose carrier was observed. The concentration of salbutamol sulphate was calculated by interpolation using the previously constructed calibration curve.

HPLC mobile phase containing the internal standard (7 ml) was introduced into the upper stage and 30 ml of the same solvent into the lower stage of a twin stage liquid impinger. The capsule, to be tested, was placed in a commercially available inhaler (either Rotahaler™, Glaxo Wellcome, Ware, UK or Cyctohater™, Pharbita BV, the Netherlands), which had been fitted into a moulded rubber mouthpiece attached to the throat piece of the impinger. Once the assembly had been checked and found to be airtight and vertical, the vacuum pump was switched on. After the pump had run for 5 s, the dose was released, the pump was allowed to run for another 7 s at 60 ± 11 min$^{-1}$ following the release of the dose and it was then switched off. The capsule shells were removed from the inhaler device and the deposition test was repeated until six capsules has been actuated in the same manner. The inhaler body, capsule shells and mouth piece were washed 5 times with the mobile phase containing internal standard and the washing solution was made up to 100 ml with the same solvent. The sample thus obtained was used to measure the amount of drug retained in the inhaler device. The same process was carried out for both the upper and the lower stage of the twin-impinger. All the samples obtained were analysed for the concentration of salbutamol sulphate using HPLC.

The recovered dose (RD) was the sum of the drug collected in the inhaler device, upper and lower stages of the impinger, whilst the emitted dose (ED) was the amount of drug released from the inhaler device, i.e. the sum of drug collected at upper and lower stages of the impinger. However, fine particle dose (FPD) was defined as the amount of drug deposited in the lower stage of the impinger, which has a diameter less than the cut-off diameter of the upper stage of a twin-impinger (6.4 µm at an air flow rate of 60 l min$^{-1}$). The fine particle fraction (FPF) was calculated as the ratio of the fine particle dose to either the recovered dose (FPF % RD) or the emitted dose (FPF % ED). The total recovery (% recovery) of the drug was assessed by the ratio of the recovered dose to the theoretical dose, the latter being the dose of salbutamol sulphate in the capsules. For example, the theoretical dose of salbutamol sulphate in one capsule was 481 ± 22 µg, which was equivalent to the filling weight (33.0 ± 1.5 mg) of lactose and salbutamol sulphate blends.

The mixtures were found to be homogenous with a coefficient of variation in salbutamol sulphate content of less than 2.2% (n = 10).

The deposition data in Table 6 were calculated as one capsule per actuation at 60 l min$^{-1}$ via a Cyclohaler™. It can be seen that the recovered dose (RD) of salbutamol sulphate varied from 391 µg for the blend containing batch 9 lactose to 508 µg for the blend composed of batch 10 lactose, corresponding to a % recovery of between 81.2-105.5%. The drug recovery was reasonably satisfactory with an average recovery of 94.1 % from all of the eight formulations investigated. The emission of drug from the inhaler device ranged from 55.6% for blends containing batch 9 lactose to 70.8% for blends containing batch 10 lactose, with an average drug emission of 66.5%, indicating that a large portion (33.5% RD) of the drug was retained in the inhaler device.

The blends containing batch 9, 10, 11 and Lactochem™ lactose produced a similar fine particle dose (FPD) of salbutamol sulphate, which was significantly higher (p < 0.01) than that obtained from the blends which were composed of batch 3, 4 or 7 lactose. The blends containing batch 9 lactose produced the highest FPF in terms of both % RD (25.6%) and % ED (46.2%), which were more than twice the FPF of the formulations containing batch 3 lactose, the FPF of the latter being 12.6% RD or 19.8% ED. These batches of lactose particles had similar particle size but with different surface smoothness and particle shape. The differences in particle shape and surface texture of lactose carrier particles may account for the differences in the deposition of the drug since all the powders are composed of the same batch of salbutamol sulphate. The lowest values for FPF of drug, obtained using blends containing batch 3 or 4 lactose may be due to those batches having the roughest surfaces with the least elongated particle shape.

TABLE 6

| Deposition of salbutamol sulphate from different batches of lactose in a twin-impinger after aerosolisation at 60 l min$^{-1}$ via a Cyclohaler™ [mean (SD), n ≥ 3]. | | | | | | | |
|---|---|---|---|---|---|---|---|
| Batch No. | RD (µg) | ED (µg) | FPD (µg) | FPF | | Recovery % | Emission % |
| | | | | % RD | % ED | | |
| *Lact | 460(20) | 320(37) | 101(12) | 21.8(1.7) | 31.6(3.5) | 95.7(4.2) | 69.3(6.0) |
| 3 | 432(18) | 276(15) | 54(10) | 12.6(2.4) | 19.8(3.9) | 89.7(3.8) | 63.8(0.9) |
| 4 | 425(24) | 294(10) | 64(2) | 15.1(0.8) | 21.8(0.7) | 88.3(5.0) | 69.1(1.7) |
| 6 | 454(20) | 319(14) | 91(8) | 20.0(1.9) | 28.5(1.9) | 94.4(4.1) | 70.2(1.9) |
| 7 | 398(28) | 257(34) | 69(18) | 17.2(3.3) | 26.6(3.6) | 82.7(5.9) | 64.6(4.0) |
| 9 | 391(48) | 217(29) | 101(18) | 25.6(1.5) | 46.2(3.8) | 81.2(10.0) | 55.6(2.5) |
| 10 | 508(13) | 359(5) | 113(5) | 22.3(1.6) | 31.5(1.9) | 105.5(2.7) | 70.8(0.8) |
| 11 | 450(35) | 344(40) | 108(7) | 21.8(2.5) | 31.9(5.4) | 103.9(7.3) | 68.7(3.7) |

*Lact = Lactochem™ lactose

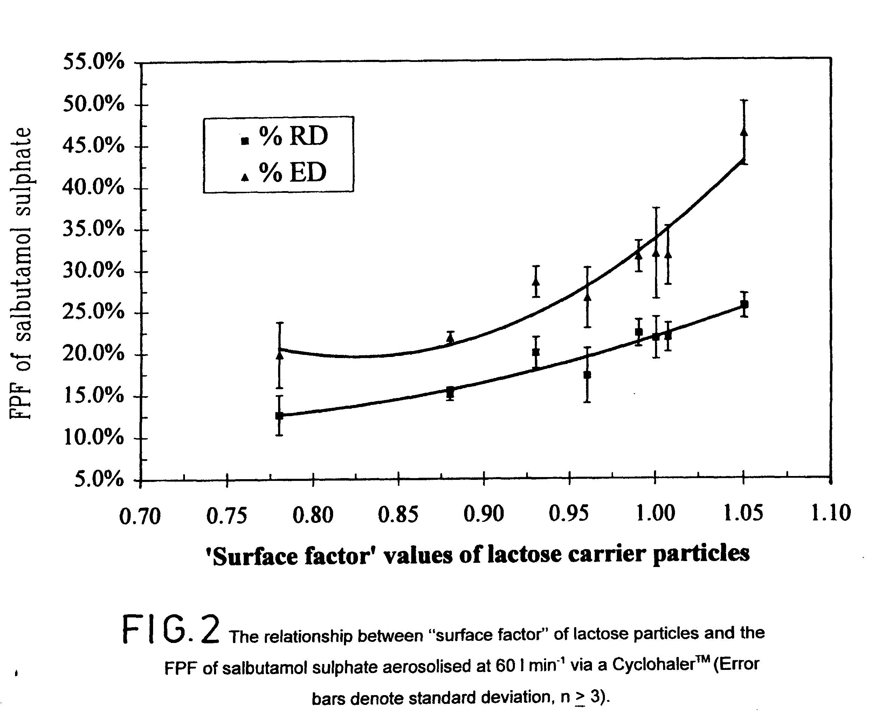

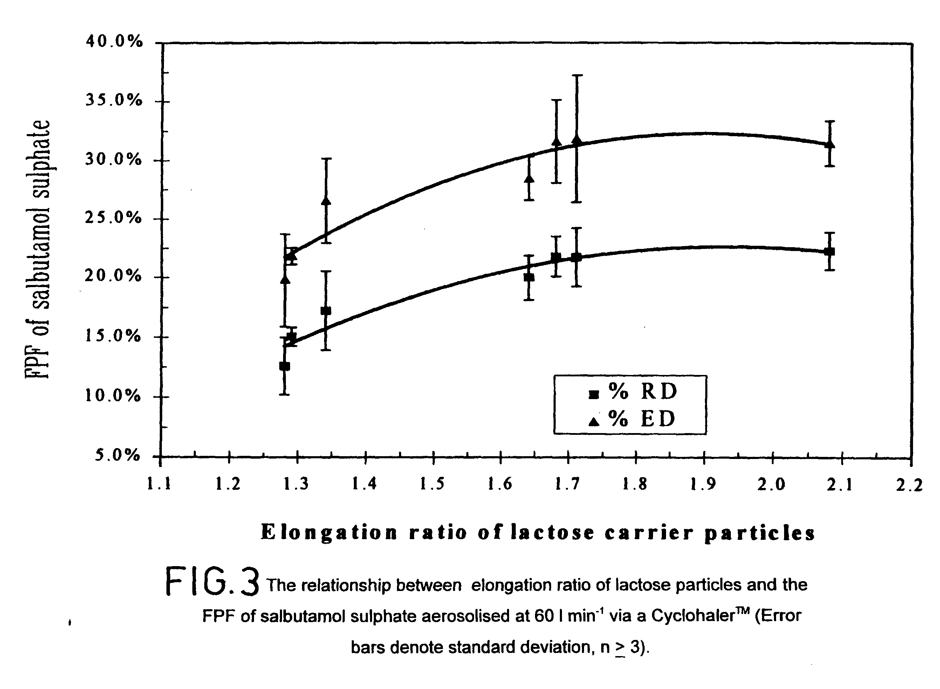

The surface smoothness and particle elongation have been quantified previously using the terms "surface factor" and elongation ratio, respectively. Figures 2 and 3 show these shape and surface descriptors of lactose carrier particles against the drug FPF of the corresponding blends.

From Figures 2 and 3, it can be seen that increasing the surface smoothness of lactose carrier particles, as expressed by the "surface factor", generally resulted in an increase in the FPF of salbutamol sulphate in terms of either % RD or % ED. Interestingly, increasing the elongation ratio of the lactose carrier particles also resulted in an increase in the FPF of salbutamol sulphate (Figure 3). These results show that apart from surface smoothness, the elongation of carrier particles may also play an important role in determining the FPF of the drug.

Example 7 - Elongated salbutamol crystals prepared by recrystallisation

Salbutamol sulphate was crystallised by adding its aqueous solution to absolute ethanol to obtain elongated crystals (needle shaped) of salbutamol sulphate having a mass median diameter of 5.49µm.

After blending with Lactochem™ lactose, the recrystallised salbutamol sulphate gave a fine particle fraction (< 6.4µm) of 22.8% recovered dose, which was more than double the fine particle fraction (10.8% recovered dose) of micronised salbutamol sulphate with a mass median diameter of 4.79µm.

These data indicate the advantage of using elongated drug particles in compositions for inhalation.

Example 8 - Elongated lactose crystals prepared by recrystallisation

Lactose Crystals (Lactochem™; Borculo Whey Ltd., Chester, UK) were sieved to produce a 63-90 µm particle size fraction. Lactose Crystals (10 g) were dissolved in 100 ml distilled water at 55 °C. After cooling to room temperature, 10 ml of this solution was transferred to a 200 ml beaker containing 90 ml of absolute ethanol which had been placed on a hot plate at 55 °C. The solution was stirred manually once to ensure better homogeneity of lactose solution and ethanol. The solution was then kept at 55 °C without disturbance. Immediately the lactose started to precipitate in large quantity from solution (usually within 10 min), the beaker was removed from the hot plate and placed at ambient temperature for 24 h. The resultant crystals were filtered through a glass filter and allowed to dry in an oven at 70 °C for 24 h. The crystals thus obtained were transferred to a sealed vial and placed in a dessicator over silica gel until required for further investigation.

The surface volume mean diameter, roundness and elongation ratio of the crystals is presented in Table 7.

Table 7:

| The surface-volume mean diameter, roundness and elongation ratio for lactose crystals (LC) and needle-shaped lactose crystals (N-S-LC) measured by an optical microscopy image analysis (n=400). | | | |
|---|---|---|---|
| Carrier (63-90 µm) | Diameter (µm) | Roundness | Elongation ratio |
| LC | 106.12 | 1.44 | 1.69 |
| N-S-LC | 68.68 | 4.24 | 6.25 |

The crystal form of the lactose particles was determined, using differential scanning calorimetry (DSC), to be α-lactose monohydrate.

Salbutamol sulphate (Allchem International, Maidenhead, UK) and lactose were mixed in a ratio of 1 : 67.5 w/w in accordance with the ratio employed in commercial Ventolin Rotacaps™. Stoppered vials, containing the separate blends of salbutamol sulphate with lactose, were placed in a Turbula mixer (Glen Greston Ltd., Middx, UK) and mixing was carried out for 30 min at 42 rev/min. All blends were then filled into hard gelatin capsules (size 3) manually such that each capsule contained $481.75 \pm 0.59$ µg salbutamol sulphate.

Deposition of salbutamol sulphate from each blend was determined using a twin-impinger after aerosolisation of 3 capsules at 60 l min$^{-1}$ via a Rotahaler. 7 ml and 30 ml respectively of the mobile phase containing the internal standard was introduced into the upper stage and lower stage of a twin stage liquid impinger. The capsule to be tested was placed in the inhaler device (Rotahafer® , Glaxo Wellcome, Ware, UK) which had been fitted into a moulded rubber mouthpiece attached to the throat piece of the impinger. Once the assembly had been checked and found to be airtight and vertical, the dose was released, the pump was switched on and allowed to run for 7s at 60 l min$^{-1}$ and then switched off. The capsule shell was then removed from the inhaler device and the deposition test was repeated so that 2 more capsules were actuated in the same manner. The capsule shells were washed 5 times with the mobile phase containing internal standard and made up to a fixed volume (50 ml). The inhaler device was washed with the same solvent and made up to volume (50 ml). The upper and lower stages of the twin stage impinger were washed individually and made up to volume (100 ml). All the samples obtained were analysed for the concentration of salbutamol sulphate.

Deposition of salbutamol sulphate from each formulation was determined at least 5 times and a variety of parameters were employed to characterised the deposition profiles of the drug. The recovered dose (RD) was the sum of the drug recovered from the capsule shells, the inhaler device, upper and lower stage of the twin impinger, whilst the emitted dose (ED) was the dose emitted from the inhaler device. Fine particle dose (FPD) was the amount of drug recovered from the lower stage (drug particles <6.4 µm) and the fine particle fraction (FPF) was calculated as the ratio of the FPD to RD. The % recovery was calculated as the ratio of RD to the theoretical dose and the % emission was defined as the ratio of ED to RD.

Table 8 shows the percentage recoveries and coefficient of variation (CV) in salbutamol sulphate content obtained for both formulations. It can be seen that both formulations showed a recovery of salbutamol sulphate close to 100% with CV less than 2%. These suggest that the overall process of mixing, sampling and analysis was accurate and reproducible, and a uniform mixing was achieved using the mixing procedure as described above.

Table 8:

| Recovery and coefficient of variation (CV) in salbutamol sulphate content obtained from the formulations containing Lactose crystals and Needle-shaped lactose crystals (n=10) | | |
|---|---|---|
| | Lactose crystals | Needle-shaped lactose crystals |
| % Recovery | 98.20 ± 1.14 | 101.78 ± 1.95 |
| % CV | 1.16 | 1.92 |

Powder formulations containing Lactose crystals and needle-shaped lactose as the carrier were shown to produce differences in the deposition of salbutamol sulphate (Tables 9 & 10). The recovered doses (RD) of salbutamol sulphate were similar for both formulations, corresponding to a percentage recovery of 93%. There was also no marked difference in the emitted dose of the drug for the formulations containing Lactose crystals and needle-shaped lactose.

The formulation containing needle-shaped lactose produced an FPD, FPF and drug dispersibility, which were 4 times higher than the formulation containing Lactose Crystals (Tables 9 & 10). The differences found in the deposition profiles of these 2 batches of lactose is likely to be attributed to the different morphological features of these lactose such as, particle, size, roundness and the elongation ratio (Table 7). Needle-shaped lactose showed a smaller particle "diameter" and a much more elongated shape, both of which may have contributed to a better dispersion of the drug in comparison to Lactose Crystals.

Table 9:

| Recovered dose (RD), emitted dose (ED) and fine particle dose (FPD) of salbutamol sulphate using Lactose crystals and Needle-shaped lactose (Mean ± SD, n=5). | | | |
|---|---|---|---|
| Carrier (63-90 µm) | RD | ED | FPD |
| Lactose Crystals | 458.6 ± 15.3 | 366.2 ± 18.8 | 25.1 ± 5.9 |
| Needle-shaped lactose | 455.8 ± 22.3 | 333.5 ± 18.7 | 100.1 ± 16.1 |

Table 10:

| Fine particle fraction, dispersibility, percentage recovery and percentage emission of salbutamol sulphate using lactose crystals and needle-shape lactose crystals (mean ± SD, n=5). | | | | |
|---|---|---|---|---|
| Carrier (63-90 µm) | FPF | Dispersibility | % Recovery | % Emission |
| Lactose Crystals | 5.5 ± 1.3 | 6.9 ± 1.6 | 93.5 ± 3.1 | 79.9 ± 5.1 |
| Needle-shaped lactose | 22.1 ± 4.3 | 29.9 ± 3.6 | 93 ± 4.6 | 73.3 ± 5.7 |

The incorporation of needle-shaped lactose produced at least 4 times the fine particle fraction and dose of salbutamol sulphate than of the formulation containing commercial grade of lactose. Therefore, the use of needle lactose has a huge potential in improving drug delivery to the lung.

Example 9 - Elongated lactose crystals prepared by recrystallisation using acetone.

Lactose Crystals (30 g; Lactochem™; Borculo Whey Ltd., Chester, UK) were dissolved in distilled water (300 ml) at 55 °C. After cooling to room temperature, the lactose solution was added to acetone without stirring according to the following proportions:

| Acetone (ml) | Lactose solution 10% (w/v) ml |
|---|---|
| 65 | 35 |
| 70 | 30 |
| 75 | 25 |
| 80 | 20 |

(continued)

| Acetone (ml) | Lactose solution 10% (w/v) ml |
|---|---|
| 85 | 15 |
| 90 | 10 |
| 95 | 5 |

Immediate precipitation was observed as the concentration of acetone increased from 80 to 95%, whereas, the remaining solutions, i.e. 75 to 65% acetone remained initially clear. The beakers containing different concentration of acetone/lactose solution were covered tightly with parafilm to ensure that no evaporation of acetone occurred during storage period, and were left unstirred for 12 h. The resultant crystals were filtered through a glass filter and allowed to dry in an oven at 55 °C for approximately 8h. The crystals thus obtained were transferred to a sealed vial and placed in a dessicator over silica gel until required for further investigation.

The surface volume mean diameter, roundness and elongation ratio of the crystals is presented in Table 11

Table 11:

| The surface-volume mean diameter, roundness and elongation ratio for lactose crystals (LC) and recrystallised lactose crystals (RLC) measured by an optical microscopy image analysis (n=400). | | | |
|---|---|---|---|
| Carrier (63-90 µm) | Diameter (µm) | Roundness | Elongation ratio |
| LC | 106.12 | 1.44 | 1.69 |
| RLC | 64.77 | 2.16 | 2.78 |

The crystalline form of the lactose particles from the 80% acetone recrystallisation was determined, using differential scanning calorimetry (DSC), to be α-lactose monohydrate.

Salbutamol sulphate and lactose were mixed in a ratio of 1 : 67.5 w/w as described in Example 8.

Deposition of salbutamol sulphate from each blend was determined as described in Example 8.

Table 12 shows the percentage recoveries and coefficient of variation (CV) in salbutamol sulphate content obtained for both formulations. It can be seen that the recovery of salbutamol sulphate is quite similar for both formulations with CV less than 3%. These suggest that the overall process of mixing, sampling and analysis was accurate and reproducible, and a uniform mixing was achieved using the mixing procedure as described above.

Table 12:

| % Recovery and coefficient of variation (CV) in salbutamol sulphate content obtained from the formulations containing Lactose crystals and recrystallised lactose obtained from 80% acetone : 20% lactose solution. (n=10). | | |
|---|---|---|
| | Lactose crystals | Recrystallised lactose |
| % Recovery | 98.20 ± 1.14 | 95.01 ± 2.50 |
| % CV | 1.16 | 2.63 |

Powder formulations containing Lactose crystals and recrystallised lactose as the carrier were shown to produce differences in the deposition of salbutamol sulphate (Tables 13 & 14). The recovered doses (RD) of salbutamol sulphate were similar for both formulations, corresponding to a percentage recovery of 93.5 % ± 3.1 and 99.4 ± 5.8 of salbutamol sulphate using lactose crystals and recrystallised lactose as a carrier respectively. Recrystallised lactose produced higher dispersibility and better emission of salbutamol sulphate from inhaler device than lactose crystals (Table 14). These suggest that recrystallised lactose has a great potential in improving the dispersion and deaggregation of salbutamol sulphate.

The formulation containing recrystallised lactose produced an FPD, FPF and drug dispersibility, which were 4 times higher than the formulation containing Lactose Crystals (Tables 13 & 14). The differences found in the deposition profiles of these 2 batches of lactose are likely to be attributed to the different morphological features of these lactose such as, particle size, roundness and the elongation ration (Table 11). Needle-shaped lactose showed a smaller particle "diameter" and more elongated shape, both of which may have contributed to a better dispersion of the drug in comparison to Lactose Crystals.

Table 13:

| Recovered dose (RD), emitted dose (ED) and final particle dose (FPD) of salbutamol sulphate using Lactose crystals and recrystallised lactose (80% acetone : 20% lactose solution). (Mean ± SD, n=5). | | | |
|---|---|---|---|
| Lactose (63-90 µm) | RD | ED | FPD |
| Lactose Crystals | 458.6 ± 15.3 | 366.2 ± 18.8 | 25.1 ± 5.9 |
| RLC (80% acetone: 20% lactose solution) | 452.1 ± 26.3 | 416.9 ± 29.2 | 100.6 ± 10.8 |

Table 14:

| Fine particle fraction, dispersibility, percentage recovery and percentage emission of salbutamol sulphate using lactose crystals and recrystallised lactose crystals (mean ± SD, n=5). | | | | |
|---|---|---|---|---|
| Lactose (63-90 µm) | FPF | Dispersibility | % Recovery | % Emission |
| Lactose Crystals | 5.5 ± 1.3 | 6.9 ± 1.6 | 93.5 ± 3.1 | 79.9 ± 5.1 |
| RLC (80% acetone: 20% lactose solution) | 22.2 ± 1.2 | 24.1 ± 0.9 | 99.4 ± 5.7 | 92.2 ± 2.8 |

## Claims

1. A pharmaceutical composition for administration via inhalation, comprising drug selected from the group consisting of salbutamol, fluticasone propionate, beclomethasone dipropionate, formotorol, budesonide, ipratropium, oxitropium or a physiologically acceptable salt or solvate thereof and lactose monohydrate crystals having an elongation ratio in the range 1.55 - 2.20.

2. A pharmaceutical composition according to claim 1 wherein the lactose monohydrate crystals have an elongation ratio is in the range 1.60 - 2.10.

3. A pharmaceutical composition according to claim 1 or 2 in which the drug is salbutamol sulphate.

4. An inhalation device comprising a pharmaceutical composition according to any one of claims 1 - 3.

5. An inhalation device according to claim 4 in which the said device is a multi-dose reservoir type.

## Patentansprüche

1. Pharmazeutische Zusammensetzung zur Verabreichung durch Inhalation, die einen Wirkstoff, der aus der Gruppe ausgewählt ist, die aus Salbutamol, Fluticasonpropionat, Beclomethasondipropionat, Formoterol, Budesonid, Ipratropium, Oxitropium oder einem physiologisch akzeptablen Salz oder Solvat davon besteht, und Lactosemonohydratkristalle mit einem Streckverhältnis im Bereich von 1,55 bis 2,20 umfasst.

2. Pharmazeutische Zusammensetzung gemäss Anspruch 1, worin die Lactosemonohydratkristalle ein Streckverhältnis im Bereich von 1,60 bis 2,10 aufweisen.

3. Pharmazeutische Zusammensetzung gemäss Anspruch 1 oder 2, worin der Wirkstoff Salbutamolsulfat ist.

4. Inhalationsvorrichtung, die eine pharmazeutische Zusammensetzung gemäss einem der Ansprüche 1 bis 3 umfasst.

5. Inhalationsvorrichtung gemäss Anspruch 4, worin die Vorrichtung ein Mehrfachdosisbehältertyp ist.

**Revendications**

1. Composition pharmaceutique pour administration par inhalation comprenant un médicament choisi dans le groupe composé de salbutamol, de fluticasone, de propionate, de dipropionate de béclométhasone, de formotorol, de budésonide, d'ipratropium, d'oxitropium ou leur sel ou solvate physiologiquement acceptable et des cristaux de monohydrate de lactose ayant un rapport d'allongement de 1,55-2,20.

2. Composition pharmaceutique selon la revendication 1, dans laquelle les cristaux de monohydrate de lactose ont un rapport d'allongement dans la gamme de 1,60-2,10.

3. Composition pharmaceutique selon la revendication 1 ou 2 dans laquelle le médicament est du sulfate de salbutamol.

4. Dispositif d'inhalation comprenant une composition pharmaceutique, selon l'une quelconque des revendications 1 à 3.

5. Dispositif d'inhalation selon la revendication 4, dans lequel ledit dispositif est de type à réservoir multi-doses.

FIG. 1.

EP 1 063 968 B1

FIG. 2 The relationship between "surface factor" of lactose particles and the FPF of salbutamol sulphate aerosolised at 60 I min$^{-1}$ via a Cyclohaler™ (Error bars denote standard deviation, n ≥ 3).

FIG.3 The relationship between elongation ratio of lactose particles and the FPF of salbutamol sulphate aerosolised at 60 l min$^{-1}$ via a Cyclohaler™ (Error bars denote standard deviation, n ≥ 3).

EP 1 063 968 B1